# DEMANDE DE BREVET EUROPEEN

(11) **EP 1 834 632 A1**
(43) Date de publication de la demande: **19.09.2007**
(21) Numéro de dépôt: 06301246.2
(22) Date de dépôt: 13.12.2006
(51) Int. Cl.: A61K 8/81, A61K 8/41, A61Q 5/12

(54) **Composition de soin et/ou de conditionnement des fibres kératiniques**

(30) Priorité: 22.12.2005 FR 0554056
(71) Demandeur: L'ORÉAL, 75008 Paris (FR)
(72) Inventeur: Parris, Eric, 92110 Clichy (FR); Fack, Géraldine, 92300 Levallois (FR)
(74) Mandataire: Le Coupanec, Pascale A.M.P.

(57) **Abrégé**

La présente invention concerne une composition de soin et/ou de conditionnement, notamment des matières kératiniques comprenant, dans un milieu physiologiquement acceptable, au moins :
- un polymère superabsorbant présent dans la composition sous forme de fibre, et
- un agent conditionneur cationique.

## Description

La présente invention concerne de nouvelles compositions cosmétiques destinées plus particulièrement au soin et/ou au conditionnement des matières kératiniques, en particulier des fibres kératiniques, notamment des cheveux.

Pour le soin et/ou le conditionnement des cheveux, l'utilisation de compositions capillaires, comprenant des agents conditionneurs cationiques est courante, notamment dans les après-shampooings.

De telles compositions capillaires sont employées dans les produits rincés ou non rincés pour apporter du soin ou du coiffant à la chevelure.

Ces compositions de base possèdent certes de bonnes qualités de soin et/ou de conditionnement mais se présentent presque toujours sous la forme d'un produit lisse plus ou moins visqueux.

La présente invention vise plus particulièrement à proposer une composition de soin et/ou de conditionnement des matières kératiniques dotée d'une texture originale comparativement à celle manifestée par les après-shampooings conventionnels.

Les inventeurs ont ainsi découvert de manière surprenante que l'utilisation de fibres de polymère(s) aux propriétés absorbantes dans des compositions d'après-shampooings permettait d'accéder à des compositions de viscosité très élevée et dotées par ailleurs d'un aspect fibreux ou cotonneux particulièrement original au regard des produits cosmétiques classiques et en particulier dans le domaine des après-shampooings. Il a été d'autre part observé que de telles compositions conservaient les propriétés de soin et/ou de conditionnement des après-shampooings classiques.

Les polymères dotés de propriétés absorbantes sont déjà utilisés notamment à des fins d'épaississement de produits cosmétiques.

Par exemple, la demande de brevet US 2003/0035783 décrit des gels de coiffage texturés par des polymères aux propriétés absorbantes.

Le document EP 1 195 157 divulgue quant à lui des compositions de produit de maquillage de la peau et des cheveux.

Cependant, dans ces documents, le polymère n'est pas à l'état fibreux et ne confère pas aux compositions de l'art antérieur l'aspect fibreux recherché dans la présente invention. De plus, ces compositions ne contiennent pas toutes un agent conditionneur cationique et ne sont pas toutes dédiées au soin et/ou au conditionnement de matières kératiniques.

Pour sa part, la demande internationale de brevet WO 03/061607 concerne des compositions, notamment de shampooing, contenant des biopolymères sous forme de fibres gélifiées à l'image des polysaccharides, comme par exemple les alginates, la gélatine et les carraghenanes. Néanmoins, ces polymères n'ont pas de propriétés absorbantes et ne sont donc pas aptes à conférer aux compositions qui les contiennent la texture fibreuse originale obtenue par la présente invention.

Ainsi, la présente invention concerne selon un premier aspect une composition de soin et/ou de conditionnement, notamment des matières kératiniques comprenant, dans un milieu physiologiquement acceptable, au moins :
- un polymère superabsorbant présent dans la composition sous forme de fibres, et
- un agent conditionneur cationique.

Selon un autre aspect, l'invention concerne l'utilisation de fibres d'un polymère superabsorbant pour la préparation d'une composition de soin et/ou de conditionnement des fibres kératiniques, notamment un après-shampooing.

La présente invention concerne également un procédé de traitement cosmétique des matières kératiniques qui comprend l'application d'une quantité efficace d'une composition telle que décrite ci-dessus sur les matières kératiniques (éventuellement mouillées) et la réalisation d'un éventuel rinçage après un éventuel temps de pose.

Ces fibres, insolubles dans le milieu, peuvent être reparties de manière uniforme ou non dans la composition. Elles sont généralement en quantité suffisante pour conférer à la composition un épaississement significatif reflété par une augmentation importante de la viscosité au niveau de la composition considérée.

Plus précisément, cette viscosité à 25 °C est supérieure à 1 Pa.s en particulier varie de 1 Pa.s à 1000 Pa.s et notamment de 10 Pa.s à 500 Pa.s et encore plus préférentiellement de 30 à 500 Pa.s à un taux de cisaillement de 1s⁻¹. Elle est appréciée de préférence à l'aide d'un viscosimètre HAAKE RS1 de THERMOELECTRON (viscosimètre à géométrie cône plan).

Dans le cadre de la présente demande, on entend par agent conditionneur tout agent ayant pour fonction l'amélioration des propriétés cosmétiques des cheveux, en particulier la douceur, la brillance, le démêlage, le toucher, le lissage, l'électricité statique.

Par « physiologiquement acceptable », on entend un milieu dénué de toxicité et compatible avec l'application sur le corps d'un être vivant, notamment d'un être humain, en particulier sur sa matière kératinique, notamment les cheveux.

Dans l'ensemble de la description, les expressions « compris entre ...et ... », « variant de ... à ... » et « allant de ... à ... » sont à comprendre dans un sens large englobant les bornes.

Outre une texture très originale et des propriétés de soin et/ou de conditionnement satisfaisantes, la viscosité élevée des compositions permet d'améliorer les propriétés d'application. La texture pâteuse du produit permet de plus une consommation au plus juste.

### POLYMERE SUPERABSORBANT

Selon la présente invention, on entend par « polymère superabsorbant », un polymère apte, lorsqu'il est à l'état sec, à absorber spontanément au moins 20 fois son poids de fluide aqueux, en particulier d'eau et notamment d'eau distillée. De tels polymères super absorbants sont décrits dans l'ouvrage « Absorbent polymer technology, Studies in polymer science 8 » de L. BRANNON-PAPPAS et R. HARLAND, édition Elsevier, 1990.

Ces polymères ont une grande capacité d'absorption et de rétention de l'eau et de fluides aqueux. Après absorption du liquide aqueux, les particules du polymère ainsi imbibées de fluide aqueux restent insolubles dans le fluide aqueux et conservent ainsi leur état particulaire individualisé.

Ainsi, le polymère superabsorbant peut posséder à l'état sec une capacité d'absorption d'eau allant de 20 à 2000 fois son propre poids (soit 20 g à 2000 g d'eau absorbée par gramme de polymère absorbant), de préférence de 30 à 1500 fois, et mieux de 50 à 1000 fois. Ces caractéristiques d'absorption d'eau sont définies aux conditions normales de température (25 °C) et de pression (760 mm Hg soit 100 000 Pa) et pour de l'eau distillée.

La valeur de la capacité d'absorption d'eau d'un polymère peut être déterminée en dispersant 0,5 g de polymère(s) dans 150 g d'une solution d'eau, en attendant 20 minutes, en filtrant la solution non absorbée sur un filtre de 150 µm pendant 20 minutes et en pesant l'eau non absorbée.

Les fibres de polymère superabsorbant ont généralement, à l'état sec, une longueur moyenne en nombre supérieure à 0,5 mm, de préférence supérieure ou égale à 1 mm, notamment allant de 1 mm à 100 mm, en particulier variant de 2 à 80 mm, voire de 3 à 60 mm, et mieux variant de 3 mm à 10 mm. On entend par longueur des fibres la plus grande dimension de ces fibres.

A l'état sec, les fibres ont généralement une largeur moyenne en nombre allant de 1 à 100 µm, en particulier de 5 à 80 µm, encore mieux de 10 à 60 µm, par exemple une largeur d'environ 30 µm. La largeur de la fibre peut correspondre à son diamètre, notamment lorsque la fibre a une section sensiblement circulaire.

Ainsi, à l'état sec, les fibres de polymère superabsorbant peuvent avoir un rapport d'aspect (longueur/largeur) allant de 50 à 10 000 (de préférence de 50 à 2500, plus préférentiellement de 75 à 1000 et encore mieux de 100 à 500, par exemple environ 200.

En présence d'eau, ces fibres gonflent.

Le polymère superabsorbant peut être partiellement ou totalement gonflé dans la composition selon l'invention.

Le polymère absorbant peut être introduit dans la composition sous forme sèche. Le polymère peut également être introduit dans un état partiellement gonflé ; pour ce faire, le polymère superabsorbant peut être mis à gonfler dans une partie du solvant, notamment de l'eau, de la composition avant d'être introduit dans la composition.

Dans la composition selon l'invention, les fibres ont généralement une largeur moyenne en nombre allant de 5 à 500 µm, de préférence 25 à 400 µm, encore mieux de 50 à 300 µm, par exemple une largeur d'environ 150 µm.

Ainsi, en passant de l'état sec à l'état mouillé, les fibres peuvent avoir une largeur augmentant d'un facteur d'environ 5.

Les fibres à l'état gonflé dans la composition peuvent avoir un rapport d'aspect allant de 1 à 2000, de préférence de 2 à 200, plus préférentiellement de 15 à 150, et encore mieux de 20 à 100, par exemple d'environ 40.

Ainsi, en passant de l'état sec à l'état mouillé, le rapport d'aspect diminue. En particulier, le rapport (rapport d'aspect à l'état gonflé/rapport d'aspect à l'état sec) peut aller de 4 à 100 %, de préférence de 8 à 75 %, encore mieux de 10 à 50 %, par exemple être d'environ 20 %.

Le polymère superabsorbant est différent d'un biopolymère et, en particulier, est un polymère synthétique. Par polymère synthétique, on entend polymère obtenu chimiquement ou par production dans un organisme des éléments nécessaires à sa production.

Comme exemple de polymères superabsorbants, on peut citer : les polymères résultants de la polymérisation avec réticulation partielle de monomères à insaturation éthylénique hydrosolubles, comme les polymères acryliques ou vinyliques, en particulier les polyacrylates réticulés et neutralisés partiellement ou totalement.

Comme polymère superabsorbant conduisant à des fibres dans les compositions de l'invention, on peut citer les produits référencés OASIS 101 3 mm ou OASIS SUB 250 MICRON ou OASIS 121/6/10 ABSORBENTS proposés par la société TECHNICAL à base d'un copolymère acrylate réticulé partiellement neutralisé et dont les longueurs respectives moyennes des fibres à l'état sec sont de l'ordre de 3 mm, 250 µm et 6 mm.

La composition selon l'invention comprend généralement le(s) polymère(s) superabsorbant(s), et notamment les fibres de polymère superabsorbant, en une teneur à l'état sec supérieure ou égale à 0,1 % en poids, par rapport à son poids total, et de préférence allant de 1 à 50 % en poids, et mieux de 3 à 40 %, voire de 5 à 25 % en poids. Avantageusement, cette quantité peut également être ajustée pour conférer une texture originale à la composition et en particulier la doter d'un aspect fibreux.

### AGENT CONDITIONNEUR CATIONIQUE

Les agents de conditionnement cationiques peuvent se présenter sous forme liquide, semi-solide ou solide.

Selon l'invention, les agents conditionneurs cationiques peuvent contenir des groupements amine primaires, secondaires, tertiaires et/ou quaternaires et peuvent être choisis parmi les polymères cationiques, les tensioactifs cationiques, les protéines cationiques, les silicones aminées et/ou leurs mélanges.

### 1) Polymères cationiques

Les polymères cationiques utilisables selon l'invention ont de préférence une densité de charge cationique inférieure à 20 meq/g, de préférence allant de 0,1 à 10 meq/g et plus particulièrement de 0,2 à 8 meq/g et encore plus particulièrement de 0,3 à 2 meq/g. La densité de charge peut être déterminée selon la méthode Kjeldahl. Elle est mesurée en général à un pH de l'ordre de 3 à 9.

Les polymères cationiques peuvent être choisis parmi tous ceux déjà connus en soi comme améliorant les propriétés cosmétiques des cheveux, à savoir notamment ceux décrits dans la demande de brevet EP-A- 0 337 354 et dans les demandes de brevets français FR-A- 2 270 846, 2 383 660, 2 598 611, 2 470 596 et 2 519 863 et ayant une densité de charge cationique telle que définie ci-dessus.

De manière encore plus générale, au sens de la présente invention, l'expression « polymère cationique » désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse molaire moyenne en nombre ou en poids comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français 2 505 348 ou 2 542 997 et leurs mélanges. Parmi ces polymères, on peut citer :
(1) les homopolymères ou copolymères dérivés d'esters ou d'amides acryliques ou méthacryliques et comportant au moins un des motifs de formules suivantes:

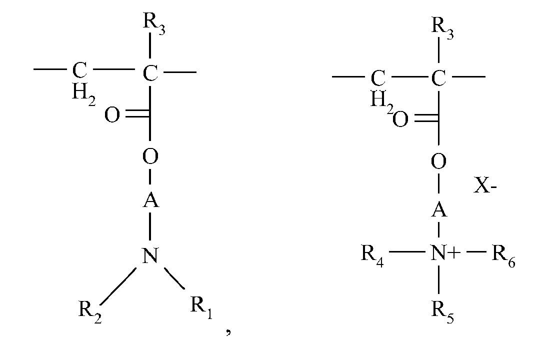

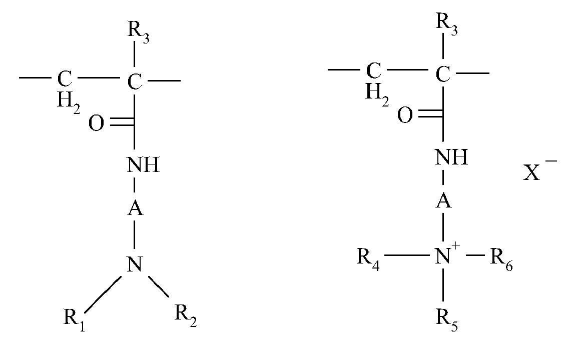

Dans lesquelles:
R₃, identiques ou différents, désignent un atome d'hydrogène ou un radical CH₃;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
R₁ et R₂, identiques ou différents, représentent hydrogène ou un groupe alkyle ayant de 1 à 6 atomes de carbone et de préférence méthyle ou éthyle ;
X désigne un anion dérivé d'un acide minéral ou organique tel que un anion méthosulfate ou un halogénure tel que chlorure ou bromure.

Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.

Ainsi, parmi ces copolymères de la famille (1), on peut citer :
- les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
- les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
- le copolymère d'acrylamide et de méthosulfate de méthacryloyloxyéthyltriméthylammonium vendu sous la dénomination RETEN par la société HERCULES,
- les copolymères vinylpyrrolidone / acrylate ou méthacrylate de dialkylaminoalkyle quaternisés ou non. Ces polymères sont décrits en détail dans les brevets français 2 077 143 et 2 393 573,
- les terpolymères méthacrylate de diméthyl amino éthyle/vinylcaprolactame/vinylpyrrolidone,
- les copolymères vinylpyrrolidone / méthacrylamidopropyl dimethylamine.
- et les copolymères vinylpyrrolidone / méthacrylamide de diméthylamino-propyle quaternisé.

(2) Les polysaccharides cationiques notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.

Les dérivés d'éthers de cellulose comportant des groupements ammonium quaternaires décrits dans le brevet français 1 492 597. Ces polymères sont également définis dans le dictionnaire CTFA comme des ammoniums quaternaires d'hydroxyéthylcellulose ayant réagi avec un époxyde substitué par un groupement triméthylammonium.

Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.

Les gommes de galactomannane cationiques sont décrites plus particulièrement dans les brevets US 3 589 578 et 4 031 307 en particulier les gommes de guar contenant des groupements cationiques trialkylammonium. On utilise par exemple des gommes de guar modifiées par un sel (par ex. chlorure) de 2,3-époxypropyl triméthylammonium.

(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361 ;

(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2 252 840 et 2 368 508 ;

(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1 583 363.

Parmi ces dérivés, on peut citer plus particulièrement les polymères acide adipique/diméthylaminohydroxypropyl/diéthylène triamine vendus sous la dénomination « CARTARETINE F, F4 ou F8 » par la société SANDOZ.

(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347.

Des polymères de ce type sont en particulier commercialisés sous la dénomination « HERCOSETT 57 » par la société Hercules Inc. par la société HERCULES dans le cas du copolymère d'acide adipique/époxypropyl/diéthylène-triamine.

(7) les co-cyclopolymères d'alkyl diallyl amine ou de dialkyl diallyl ammonium tels que les copolymères comportant comme constituant principal de la chaîne des motifs répondant aux formules (I) ou (I') : 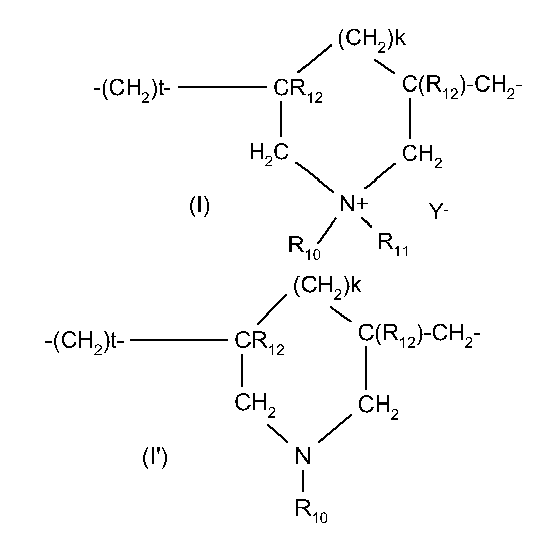 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄) ou R₁₀ et R₁₁ peuvent désigner conjointement avec l'atome d'azote auquel ils sont rattachés, des groupement hétérocycliques, tels que pipéridinyle ou morpholinyle ; Y- est un anion tel que bromure, chlorure, acétate, borate, citrate, tartrate, bisulfate, bisulfite, sulfate, phosphate. Ces polymères sont notamment décrits dans le brevet français 2 080 759 et dans son certificat d'addition 2 190 406.

Selon un mode de réalisation particulier, R₁₀ et R₁₁, indépendamment l'un de l'autre, désignent un groupement alkyle ayant de 1 à 4 atomes de carbone.

Parmi les polymères définis ci-dessus, on peut citer plus particulièrement les copolymères de chlorure de diallyldiméthylammonium et d'acrylamide vendu sous la dénomination « MERQUAT 550 » par la société NALCO.

(8) les polymères de diammonium quaternaire contenant des motifs récurrents répondant à la formule :

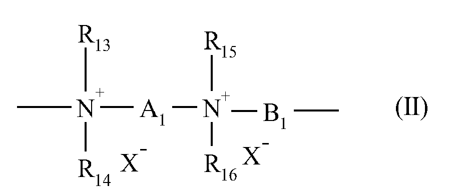

Formule (II) dans laquelle :
R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C₁-C₆ linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X- désigne un anion dérivé d'un acide minéral ou organique;
A₁, R₁₃ et R₁₅ peuvent former avec les deux atomes d'azote auxquels ils sont rattachés un cycle pipérazinique ; en outre si A₁ désigne un radical alkylène ou hydroxyalkylène linéaire ou ramifié, saturé ou insaturé, B₁ peut également désigner un groupement (CH₂)n-CO-D-OC-(CH₂)n-
dans lequel D désigne :
a) un reste de glycol de formule : -O-Z-O-, où Z désigne un radical hydrocarboné linéaire ou ramifié ou un groupement répondant à l'une des formules suivantes :

   -(CH₂-CH₂-0) ₓ-CH₂-CH₂-

   -[CH₂-CH (CH₃)-O]_{y}-CH₂-CH (CH₃)-

   Où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

   -CH₂-CH₂-S-S-CH₂-CH₂- ;
d) un groupement uréylène de formule : -NH-CO-NH- ;

De préférence, X- est un anion tel que le chlorure ou le bromure.

Ces polymères ont une masse molaire moyenne en nombre généralement comprise entre 1 000 et 100 000.

Des polymères de ce type sont notamment décrits dans les brevets français 2 320 330, 2 270 846, 2 316 271, 2 336 434 et 2 413 907 et les brevets US 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020.

On peut utiliser plus particulièrement les polymères qui sont constitués de motifs récurrents répondant à la formule : 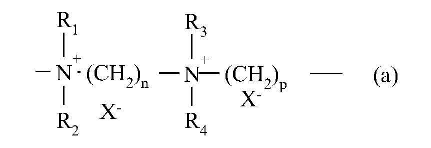 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.

Un composé de formule (a) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).

(9) les polymères de polyammonium quaternaires comprenant des motifs de formule (III): 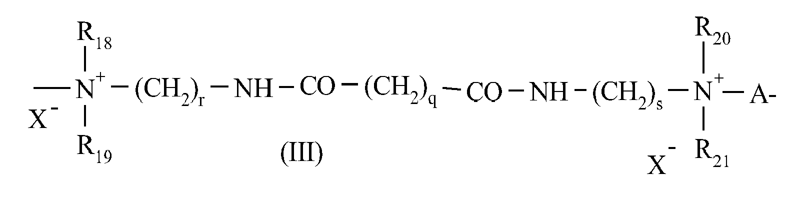 formule dans laquelle :
- R₁₈, R₁₉, R₂₀ et R₂₁, identiques ou différents, représentent un atome d'hydrogène ou un radical méthyle, éthyle, propyle, (β-hydroxyéthyle, (β-hydroxypropyle ou -CH₂CH₂(OCH₂CH₂)ₚOH, où p est égal à 0 ou à un nombre entier compris entre 1 et 6, sous réserve que R₁₈, R₁₉, R₂₀ et R₂₁ ne représentent pas simultanément un atome d'hydrogène,
- r et s, identiques ou différents, sont des nombres entiers compris entre 1 et 6,
- q est égal à 0 ou à un nombre entier compris entre 1 et 34,
- X- désigne un anion tel qu'un halogènure,
- A désigne un radical d'un dihalogénure ou représente de préférence -CH₂-CH₂-O-CH₂-CH₂-.

De tels composés sont notamment décrits dans la demande de brevet EP-A-122 324.

On peut par exemple citer parmi ceux-ci, les produits « Mirapol^{®} A 15 », « Mirapol^{®} AD1 », « Mirapol^{®} AZ1 » et « Mirapol^{®} 175 » vendus par la société Miranol.

(10) Les polymères quaternaires de vinylpyrrolidone et de vinylimidazole tels que par exemple les produits commercialisés sous les dénominations Luviquat^{®} FC 905, FC 550 et FC 370 par la société B.A.S.F.

(11) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine, notamment les chitosanes ou leurs sels ;

Les sels utilisables sont en particulier l'acétate, le lactate, le glutamate, le gluconate ou le pyrrolidone-carboxylate de chitosane.

Parmi ces composés, on peut citer le chitosane ayant un taux de désacétylation de 90 % en poids, le pyrrolidone-carboxylate de chitosane commercialisé sous la dénomination KYTAMER^{®} PC par la société AMERCHOL.

Parmi tous les polymères cationiques susceptibles d'être utilisés dans le cadre de la présente invention, on préfère mettre en oeuvre, les dérivés d'éther de cellulose quaternaires tels que les produits vendus sous la dénomination « JR 400 » par la Société AMERCHOL, les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société NALCO, les polymères quaternaires de vinylpyrrolidone et de vinylimidazole, les homopolymères ou copolymères réticulés de sels de méthacryloyloxyalkyl(C₁-C₄) trialkyl(C₁-C₄)ammonium, le pyrrolidone-carboxylate de chitosane commercialisé sous la dénomination KYTAMER^{®} PC par la société AMERCHOL et leurs mélanges.

### 2) Tensioactifs cationiques

Les tensioactifs cationiques utilisables selon la présente invention sont notamment les sels d'amines grasses primaires, secondaires ou tertiaires, éventuellement polyoxyalkylénées ; les sels d'ammonium quaternaire ; les dérivés d'imidazoline ; les oxydes d'amines à caractère cationique, et/ou l'un de leurs mélanges.

Les sels d'ammonium quaternaires sont par exemple :
- ceux qui présentent la formule générale (IV) suivante: 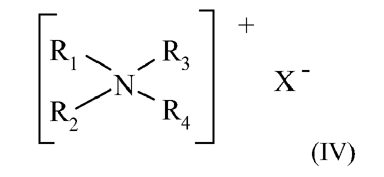
dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone; X est un anion choisi dans le groupe des halogénures, phosphates, acétates, lactates, alkyl(C₂-C₆)sulfates, alkyl-ou-alkylarylsulfonates, De préférence R₁ et R₂ désigne un alkyle en C₁-C₄, ou un hydroxyalkyle en C₁-C₄.
- les sels d'ammonium quaternaire de l'imidazolinium, comme par exemple celui de formule (V) suivante : 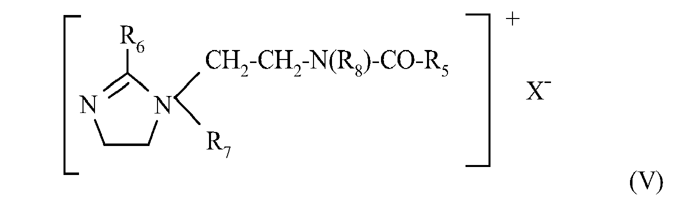
dans laquelle R₅ représente un radical alcényle ou alkyle comportant de 8 à 30 atomes de carbone par exemple dérivés des acides gras de coprah, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X- est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène.
- les sels de diammonium quaternaire de formule (VI) : 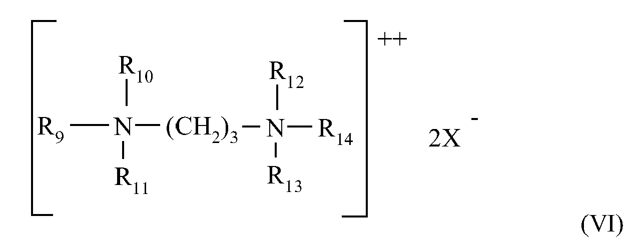
dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates.
- les sels d'ammonium quaternaire contenant au moins une fonction ester par exemple ceux de formule (VII) suivante : 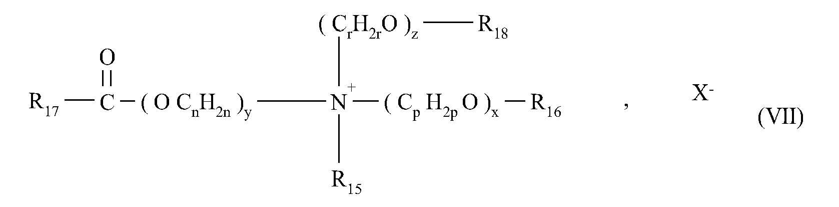
dans laquelle :
- R₁₅ est choisi parmi les radicaux alkyles en C₁-C₆ et les radicaux hydroxyalkyles ou dihydroxyalkyles en C₁-C₆ ;
- R₁₆ est choisi parmi :

- le radical 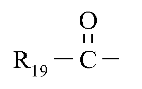
- les radicaux R₂₀ hydrocarbonés en C₁-C₂₂ linéaires ou ramifiés, saturés ou insaturés,
- l'atome d'hydrogène,
- R₁₈ est choisi parmi :

- le radical 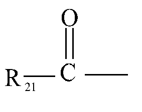
- les radicaux R₂₂ hydrocarbonés en C₁-C₆ linéaires ou ramifiés, saturés ou insaturés,
- l'atome d'hydrogène,
- R₁₇, R₁₉ et R₂₁, identiques ou différents, sont choisis parmi les radicaux hydrocarbonés en C₇-C₂₁, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X- est un anion simple ou complexe, organique ou inorganique ;

Sous réserve que la somme x + y + z vaut de 1 à 15, que lorsque x vaut 0 alors R₁₆ désigne R₂₀ et que lorsque z vaut 0 alors R₁₈ désigne R₂₂.

Les radicaux alkyles R₁₅ peuvent être linéaires ou ramifiés et plus particulièrement linéaires.

De préférence R₁₅ désigne un radical méthyle, éthyle, hydroxyéthyle ou dihydroxypropyle et plus particulièrement un radical méthyle ou éthyle.

Avantageusement, la somme x + y + z vaut de 1 à 10.

Lorsque R₁₆ est un radical R₂₀ hydrocarboné, il peut être long et avoir de 12 à 22 atomes de carbone ou court et avoir de 1 à 3 atomes de carbone.

Lorsque R₁₈ est un radical R₂₂ hydrocarboné, il a de préférence 1 à 3 atomes de carbone.

Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁₋C₂₁, linéaires ou ramifiés, saturés ou insaturés.

De préférence, x et z, identiques ou différents, valent 0 ou 1.

Avantageusement, y est égal à 1.

De préférence, n, p et r, identiques ou différents, valent 2 ou 3 et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X- est encore plus particulièrement le chlorure ou le méthylsulfate.

On utilise plus particulièrement les sels d'ammonium de formule (VII) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :

- le radical 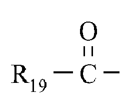
- les radicaux méthyle, éthyle ou hydrocarbonés en C₁₄-C₂₂
- l'atome d'hydrogène ;
- R₁ 18 est choisi parmi :

- le radical 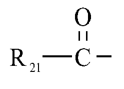
- l'atome d'hydrogène ;

R₁₇, R₁₉ et R₂₁, identiques ou différents, sont choisis parmi les radicaux hydrocarbonés en C₁₃-C₁₇, linéaires ou ramifiés, saturés ou insaturés et de préférence parmi les radicaux alkyles et alcényle en C₁₃-C₁₇, linéaires ou ramifiés, saturés ou insaturés.

Avantageusement, les radicaux hydrocarbonés sont linéaires.

Parmi les sels d'ammonium quaternaire de formule (IV) on préfère, d'une part, les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyldiméthylstéaryl ammonium ou encore, d'autre part, le chlorure de palmitylamidopropyltriméthylammonium ou le chlorure de stéaramidopropyldiméthyl (myristyl acétate) ammonium commercialisé sous la dénomination « CERAPHYL 70 » par la société VAN DYK.

On peut citer par exemple les composés de formule (V) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus par exemple par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART par la société COGNIS, STEPANQUAT par la société STEPAN, NOXAMIUM par la société CECA, REWOQUAT WE 18 et REWOQUAT W75 par la société DEGUSSA.

On peut aussi utiliser les sels d'ammonium contenant au moins une fonction ester décrits dans les brevets US-A-4874554 et US-A-4137180.

Des sels de diammonium quaternaire de formule (VI) convenant à l'invention comprennent notamment le dichlorure de propanesuif diammonium.

### 3) Protéines cationiques

Les compositions selon l'invention peuvent comprendre des protéines cationiques ou des hydrolysats de protéines cationiques qui sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :
- les hydrolysats de collagène portant des groupements triéthylammonium tels que les produits vendus sous la dénomination « Quat-Pro E » par la Société MAYBROOK et dénommés dans le dictionnaire CTFA « Triéthonium Hydrolyzed Collagen Ethosulfate » ;
- les hydrolysats de collagène portant des groupements chlorure de triméthylammonium et de triméthylstéarylammonium, vendus sous la dénomination de « Quat-Pro S » par la Société MAYBROOOK et dénommés dans le dictionnaire CTFA « Steartrimonium Hydrolyzed Collagen » ;
- les hydrolysats de protéines animales portant des groupements triméthylbenzylammonium tels que les produits vendus sous la dénomination « Crotein BTA » par la Société CRODA et dénommés dans le dictionnaire CTFA « Benzyltrimonium hydrolyzed animal protein » ;
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres :
- le « Croquat L » dont les groupements ammonium quaternaires comportent un groupement alkyle en C₁₂ ;
- le « Croquat M » dont les groupements ammonium quaternairees comportent des groupements alkyles en C₁₀-C₁₈ ;
- le « Croquat S » dont les groupements ammonium quaternaires comportent un groupement alkyle en C₁₈ ;
- le « Crotein Q » dont les groupements ammonium quaternaires comportent au moins un groupe alkyle ayant de 1 à 18 atomes de carbone.

Ces différents produits sont vendus par la Société Croda.

D'autre protéines ou hydrolysats quaternisés sont par exemple ceux répondant à la formule (VIII) : 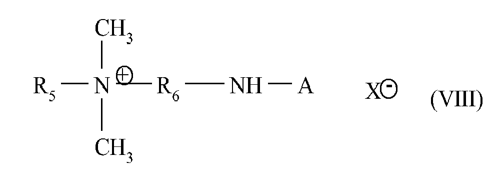 dans laquelle X- est un anion d'un acide organique ou minéral, A désigne un reste de protéine dérivé d'hydrolysats de protéine de collagène, R₅ désigne un groupement lipophile comportant jusqu'à 30 atomes de carbone, R₆ représente un groupement alkylène ayant 1 à 6 atomes de carbone. On peut citer par exemple les produits vendus par la Société Inolex, sous la dénomination « Lexein QX 3000 », appelé dans le dictionnaire CTFA « Cocotrimonium Collagent Hydrolysate ».

On peut encore citer les protéines végétales quaternisées telles que les protéines de blé, de maïs ou de soja : comme protéines de blé quaternisées, on peut citer celles commercialisées par la Société Croda sous les dénominations « Hydrotriticum WQ ou QM », appelées dans le dictionnaire CTFA « Cocodimonium Hydrolysed wheat protein », « Hydrotriticum QL » appelée dans le dictionnaire CTFA « Laurdimonium hydrolysed wheat protein », ou encore « Hydrotriticum QS », appelée dans le dictionnaire CTFA « Steardimonium hydrolysed wheat protein ».

### 4) Silicone aminée

Selon l'invention, on désigne par silicone aminée toute silicone comportant au moins une amine primaire, secondaire, tertiaire ou un groupement ammonium quaternaire. Les silicones aminées sont par exemple choisies parmi :

### a) les polysiloxanes dénommés dans le dictionnaire CTFA « amodiméthicone » et répondant à la formule :

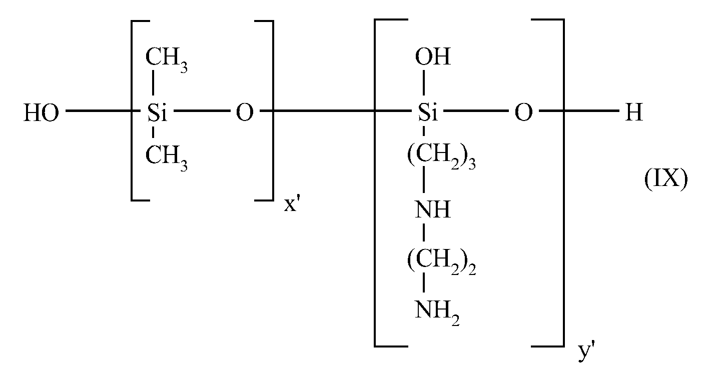

dans laquelle x' et y' sont des nombres entiers dépendant du poids moléculaire, généralement tels que ledit poids moléculaire moyen en poids est compris entre 5 000 et 500 000 environ ;

### b) les silicones aminées répondant à la formule :

R_{'a}G₃₋ₐ-Si(OSiG₂)ₙ-(OSiG_{b}R'₂-_{b})m-O-SiG₃₋ₐ₋R'ₐ (X)

dans laquelle :
G est un atome d'hydrogène, ou un groupement phényle, OH, ou alkyle en C₁-C₈, par exemple méthyle,
a désigne le nombre 0 ou un nombre entier de 1 à 3, en particulier 0,
b désigne 0 ou 1, et en particulier 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10;
R' est un radical monovalent de formule -CqH2qL dans laquelle q est un nombre de 2 à 8 et L est un groupement aminé éventuellement quaternisé choisi parmi les groupements :
   -NR"-Q-N'(R")₂
   -N(R")₂
   -N⊕(R")₃ A⁻
   -NH⊕(R")₂ A⁻
   -NH₂⊕(R") A⁻
   -N(R")-Q-N⊕R"H₂ A⁻
   -NR"-Q-N⊕ (R")₂H A⁻
   -NR"-Q-NE⊕(R")₃ A⁻,
   dans lesquels R" peut désigner hydrogène, phényle, benzyle, ou un radical hydrocarboné saturé monovalent, par exemple un radical alkyle ayant de 1 à 20 atomes de carbone ; Q désigne un groupement de formule CᵣH₂ᵣ, linéaire ou ramifié, r étant un entier allant de 2 à 6, de préférence de 2 à 4 ; et A- représente un ion halogénure tel que par exemple fluorure, chlorure, bromure ou iodure.
   Un produit correspondant à cette définition est la silicone dénommée « triméthylsilylamodiméthicone », répondant à la formule : 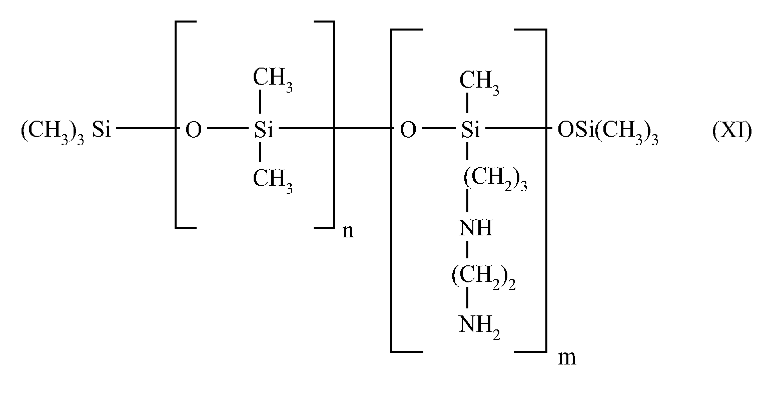 dans laquelle n et m ont les significations données ci-dessus (cf formule X).
   De tels polymères sont décrits par exemple dans la demande de brevet EP-A-95 238.

### c) les silicones aminées répondant à la formule :

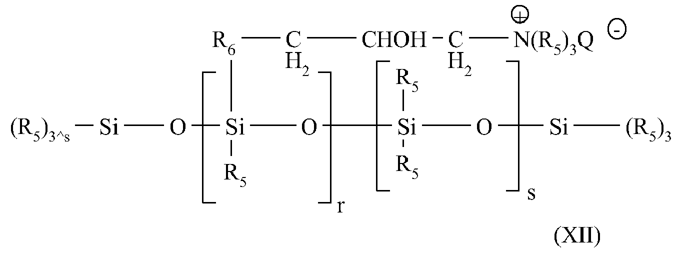

dans laquelle :
R₅ représente un radical hydrocarboné monovalent ayant de 1 à 18 atomes de carbone, et en particulier un radical alkyle en C₁-C₁₈, ou alcényle en C₂-C₁₈, par exemple méthyle ;
R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
Q- est un anion tel qu'un ion halogénure, notamment chlorure ou un sel d'acide organique (acétate ...) ;
r représente une valeur statistique moyenne de 2 à 20 et en particulier de 2 à 8 ;
s représente une valeur statistique moyenne de 20 à 200 et en particulier de 20 à 50.

De telles silicones aminées sont décrites plus particulièrement dans le brevet US 4 185 087.

### d) les silicones ammonium quaternaire de formules XIII, XIV, XV, XVI ou XVII suivantes :

- les silicones ammonium quaternaire de formules XIII suivante : 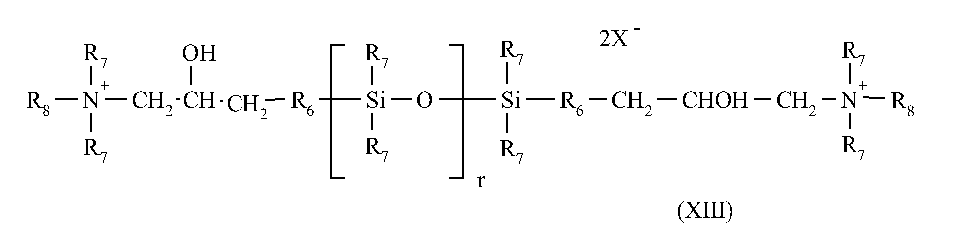
dans laquelle :
R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle
R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈, un radical -R₆-NHCOR₇ ;
X- est un anion tel qu'un ion halogénure, notamment chlorure ou un sel d'acide organique (acétate ...);
r représente une valeur statistique moyenne de 2 à 200 et en particulier de 5 à 100 ;

Ces silicones sont par exemple décrites dans la demande EP-A-0530974.

Les silicones ammonium quaternaire de formules XIV, XV, XVI ou XVII suivantes : 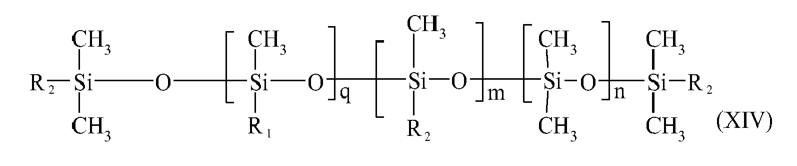 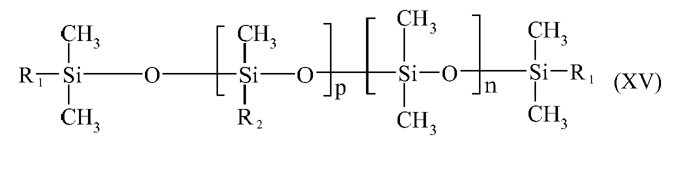 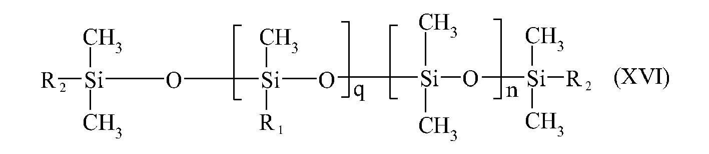 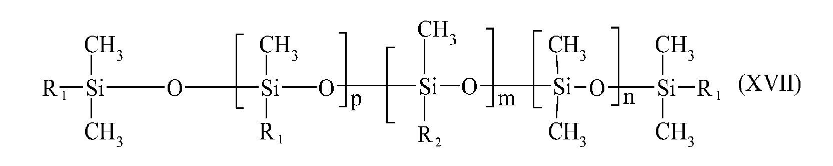 formules dans lesquelles :
- R₁, identique ou différent, représente un groupe alkyle, linéaire ou ramifié, en C₁-C₃₀ ou phényle ;
- R₂, identique ou différent, représente -C_{c}H_{2c}-0-(C₂H₄0)ₐ-(C₃H₆0)_{b}-R₅ ou -C_{c}H_{2c}-O-(C₄H₈)ₐ-R₅;
- R₃ et R₄, identiques ou différents, désignent chacun un groupe alkyle, linéaire ou ramifié, en C₁-C₁₂, et de préférence un groupe méthyle ;

R₅, identique ou différent, est choisi parmi les groupes de formule suivante : 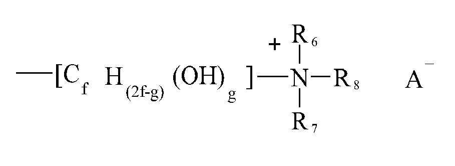
- les radicaux R₈ représentent indépendamment un radical alkyle en C₁₋₂₂ ou alcényle en C₂₋₂₂, linéaire ou ramifié, et portant éventuellement un ou plusieurs

groupements OH, ou représentent un groupement CₕH₂ₕZCOR₉ ;
- R₆, R₇ et R₉, identiques ou différents, représentent des radicaux alkyle en C₁-22 ou alcényle en C₂₋₂₂, linéaires ou ramifiés, portant éventuellement un ou plusieurs groupements OH, ou R₇ peut former avec une partie de R₈ un hétérocycle (cycle avec au moins un hétéroatome tel que par exemple N, O, P), l'hétérocycle est notamment une imidazoline. De préférence, R₆ et R₇ désignent un radical alkyle en C₁-C₆ et plus particulièrement un méthyle, R₉ désigne de préférence un radical choisi parmi les alkyle en C₈-C₁₈ et les alcényl en C₈-C₁₈ et notamment un radical cocoyle,
- m varie de 0 à 20,
- n varie de 0 à 500,
- p varie de 1 à 50,
- q varie de 0 à 20,
- a varie de 0 à 50,
- b varie de 0 à 50,
- c varie de 0 à 4,
- f varie de 0 à 4,
- g varie de 0 à 2, de préférence est égal à 1,
- h varie de 1 à 4, de préférence est égal à 3,
- Z représente un atome d'oxygène ou NH,
- A- représente un anion minéral ou organique monovalent tel qu'un halogénure (chlorure, bromure), un sulfate, ou un carboxylate (acétate, lactate, citrate).

De préférence, on utilise les silicones à ammonium quaternaire répondant à la formule générale (XVI) telle que définie ci-dessus, et plus particulièrement, ceux répondant à la formule générale (XVI) dans laquelle au moins l'une des, et de préférence toutes les conditions suivantes sont satisfaites :
- c est égal à 2 ou 3,
- R₁ désigne un groupe méthyle,
- a et b sont égaux à zéro,
- n varie de 0 à 100,
- q est égal à 0,
- f = 3,
- g= 1,
- R₆ et R₇ désignent le groupe méthyle,
- R₈ désigne le radical -(CH₂)-NHCOR₉.

De telles silicones sont commercialisées par exemple par la société GOLDSCHMIDT sous les dénominations ABIL QUAT 3272, ABIL B 9905, ABIL QUAT 3474 et ABIL K 3270, par la société LIPO France sous les dénominations SILQUAT Q-100, SILQUAT Q-200 WS, SILQUAT AX, SILQUAT AC, SILQUAT AD et SILQUAT AM tous fabriqués par la société SILTECH, par la société OSI sous la dénomination MAGNASOFT EXHAUST et SILSOFT C-880 et par la société UCIB sous les dénominations PECOSIL 14-PQ et PECOSIL 36-PQ (fabricant PHOENIX CHEMICAL).

Ces silicones sont notamment décrites dans les brevets EP 530 974, DE 3 719 086, DE 3 705 121, EP 617 607 et EP 714 654.

Les silicones à groupements ammonium quaternaire utilisées conformément à l'invention peuvent se présenter sous forme de solutions aqueuses ou éventuellement sous forme de dispersions ou d'émulsions dans l'eau.

### e) les silicones aminées de formule (XVIII) :

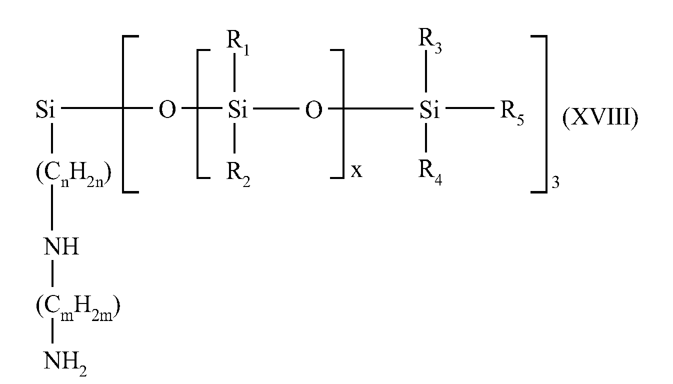

dans laquelle :
- R₁, R₂, R₃ et R₄, identiques ou différents, désignent un radical alkyle en C₁-C₄ ou un groupement phényle,
- R₅ désigne un radical alkyle en C₁-C₄ ou un groupement hydroxyle,
- n est un entier variant de 1 à 5,
- m est un entier variant de 1 à 5,
   et dans laquelle x est choisi de manière telle que l'indice d'amine soit compris entre 0,01 et 1 meq/g.

Les silicones particulièrement préférées conformément à l'invention sont les polysiloxanes à groupements aminés tels que les amodiméthicones ou les triméthylsilylamodiméthicones (CTFA 4ème édition 1997), et encore plus particulièrement les silicones à groupements ammonium quaternaire.

Il est bien entendu possible de mettre en oeuvre des mélanges d'agents conditionneur.

La quantité et la qualité des agents conditionneurs cationiques sont ajustées pour conférer à la composition finale des qualités de soin et/ou de conditionnement satisfaisantes.

Ainsi, selon l'invention, les compositions peuvent comprendre 0,1 % en poids ou plus d'agent(s) conditionneur(s) cationique(s) et notamment peuvent comprendre de 0,2 % à 10 % en poids, de préférence de 0,3 % à 8 % en poids, et encore plus préférentiellement de 0,4 % à 6 % en poids d'agent(s) conditionneur(s) cationique(s).

### PARTICULES

Dans un mode de réalisation particulier, les compositions de lavage de l'invention peuvent comprendre en outre des particules insolubles différentes des fibres de polymères absorbants.

La présence de particules insolubles permet notamment de prolonger la conservation dans le temps de l'aspect fibreux des compositions voire d'accroître l'aspect fibreux au niveau de la composition.

Ces particules peuvent être minérales ou organiques.

Les particules organiques peuvent notamment être choisies parmi les particules de verre, de polyamides tels que le Nylon, de polymères ou copolymères d'acrylonitrile, de chlorure de vinylidène, de chlorure de vinyle et/ou de monomère acrylique ou styrénique, éventuellement expansés. Le monomère acrylique est par exemple un acrylate ou méthacrylate de méthyle ou d'éthyle. Le monomère styrénique est par exemple l'α-méthyl-styrène ou le styrène.

Les particules minérales comprennent de préférence au moins un élément des colonnes IIa, IIIa, IVa, IIIb et IVb du tableau de classification périodique des éléments et de préférence des colonnes IIa, IIIa et IVa. Elles sont notamment choisies parmi les particules contenant au moins 10 % en poids de carbonate de calcium ou d'au moins un silicate, les particules contenant au moins 90 % d'oxyde d'aluminium, les silices, l'oxyde de magnésium, les oxydes de Zinc, les oxydes de titane, le sulfate de baryum, le nitrure de Bore et leurs mélanges.

Les silicates utilisables selon l'invention peuvent être choisis parmi les silicates de sodium, de magnésium tel que le talc et/ou de lithium, On peut notamment utiliser les composés commercialisés par la société LAPORTE sous la dénomination LAPONITE XLG et LAPONITE XLS.

Les silicates convenant dans les compositions de la présente invention peuvent être d'origine naturelle ou d'origine synthétique.

Les particules solides présentent, de préférence, une taille primaire moyenne en nombre comprise entre 2 nm et 2 microns, plus préférentiellement entre 5nm et 500 nm, et plus préférentiellement encore entre 10 nm et 250 nm. Au sens de la présente invention, on entend par « taille primaire de particules », la dimension maximale qu'il est possible de mesurer entre deux points diamétralement opposés d'une particule individuelle.

Les particules selon l'invention peuvent, par exemple, avoir une forme quelconque, par exemple la forme de sphères, de paillettes, d'aiguilles, de plaquettes ou des formes totalement aléatoires. De préférence, elles sont sous forme de plaquettes.

Selon l'invention, les compositions peuvent comprendre de 0,001 % à 20 % en poids, de préférence de 0,01 % à 10 % en poids et plus particulièrement de 0,1 à 3 % en poids de particules insolubles.

Le rapport pondéral particules/fibres peut varier de 0,2 à 10, en particulier de 0,5 à 1,2.

Selon un mode particulier de l'invention, la composition comprend des fibres de copolymère acrylate réticulé, notamment une polyacrylate de sodium et des plaquettes de talc et/ou de nitrure de Bore.

### MILIEU PHYSIOLOGIQUEMENT ACCEPTABLE

Le milieu physiologiquement acceptable comprend préférentiellement de l'eau.

La composition peut comprendre, un milieu hydrophile comprenant de l'eau ou un mélange d'eau et de solvant(s) organique(s) hydrophile(s) comme les alcools et notamment les monoalcools, linéaires ou ramifiés en C₁-C₆, comme l'éthanol, le tertiobutanol, le n-butanol, l'isopropanol ou le n-propanol, et les polyols comme la glycérine, le propylène glycol, le sorbitol, ou bien encore les éthers de polyols.

L'eau ou le mélange d'eau et de solvants organiques hydrophiles peut être présent dans la composition selon l'invention en une teneur allant de 30 % à 99 % en poids, par rapport au poids total de la composition, et de préférence de 50 % à 95 % en poids.

De préférence, la composition comprend plus de 30 %, voire de 50 à 95 % en poids d'eau par rapport au poids total de la composition.

Les compositions de soin et/ou de conditionnement selon l'invention présentent un pH final généralement compris entre 2 et 11. De préférence, ce pH est compris entre 3 et 10, voire entre 4 et 8. Il peut être ajusté à la valeur désirée au moyen d'agents acidifiants ou alcalinisants bien connus de l'état de la technique des compositions appliquées sur des matières kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule (XIX) suivante :

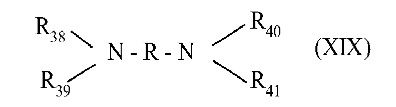

Dans laquelle R est un reste propylène éventuellement substitué par un groupement hydroxyle ou un radical alkyle en C₁ - C4 ; R38, R39, R40 et R41, identique ou différent, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

### AUTRES COMPOSES

Les compositions conformes à l'invention peuvent contenir en plus des polymères superabsorbants d'eau d'autres composés régulateurs de viscosité tels que des électrolytes, ou des agents épaississants (associatifs ou non associatifs). On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination « AMINOL A15 » par la société CHEM Y, les acides polyacryliques réticulés et les copolymères d'acide acrylique tels que les copolymères d'acide acrylique et d'acrylates d'alkyle en C₁₀-C₃₀ réticulés, à l'état non fibreux. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir de préférence jusqu'à 5 % d'agents nacrants ou opacifiants bien connus dans l'état de la technique tels que par exemple les alcools gras supérieurs à C₁₆, les dérivés acylés à chaîne grasse tels que les monostéarates ou distéarates d'éthylène glycol ou de polyéthylèneglycol, les éthers à chaînes grasses (C₁₀-C₃₀) tels que par exemple le distéaryléther ou le 1-(hexadécyloxy)-2-octadécanol. 1.

Les compositions conformes à l'invention peuvent éventuellement contenir en outre au moins un additif choisi parmi les synergistes de mousses tels que des 1,2-alcanediols en C₁₀-C₁₈ ou des alcanolamides gras dérivés de mono ou de diéthanolamine, les filtres solaires siliconés ou non, les polymères anioniques ou non ioniques, les protéines, les hydrolysats de protéines, les céramides, les pseudocéramides, les acides gras à chaînes linéaires ou ramifiées en C₁₂-C₄₀ tels que l'acide méthyl-18 eicosanoique, les hydroxyacides, les vitamines, les provitamines tels que le panthénol, les huiles animales, minérales ou de synthèse et tout autre additif classiquement utilisé dans le domaine cosmétique qui n'affecte pas les propriétés des compositions selon l'invention.

Les compositions de soin et/ou de conditionnement selon l'invention peuvent bien entendu contenir en outre tous les adjuvants usuels rencontrés dans le domaine des après-shampooings, comme par exemple des parfums, des agents conservateurs, des sequestrants, des adoucissants, des colorants, des agents hydratants, des agents anti-pélliculaires ou anti-séborrhéiques, et autres.

Les compositions conformes à l'invention peuvent éventuellement contenir en outre d'autres agents ayant pour effet d'améliorer les propriétés cosmétiques de cheveux ou de la peau sans cependant altérer la stabilité des compositions. On peut citer à ce sujet les polymères anioniques ou non ioniques ou amphotères, les protéines, les hydrolysats de protéines, les cires naturelles ou synthétiques, les céramides, les pseudocéramides, les acides gras à chaînes linéaires ou ramifiées en C₁₆-C₄₀ tels que l'acide méthyl-18 eicosanoique, les esters gras d'acides carboxyliques, les mono-, di- ou triglycérides d'acides gras, les vitamines, les provitamines telles que le panthénol, les huiles végétales, les huiles de synthèses telles que les poly-alphaoléfmes, les huiles fluorées ou perfluorées, et leurs mélanges.

Les compositions selon l'invention peuvent être exemptes de carboxyméthyl cellulose.

Les compositions selon l'invention comprennent de préférence au moins une silicone différentes des silicones aminées.

A titre de silicones utilisables dans les compositions de la présente invention, on peut notamment citer les silicones volatiles ou non, cycliques ou acycliques, ramifiées ou non, organomodifiées ou non, telles que décrites ci-dessous.

Les silicones utilisables conformément à l'invention peuvent être solubles ou insolubles dans la composition et en particulier être des polyorganosiloxanes insolubles dans la composition de l'invention ; elles peuvent se présenter sous forme d'huiles, de cires, de résines ou de gommes.

Selon l'invention, toutes les silicones peuvent être utilisées telle quelle ou sous formede solutions, de dispersions, d'émulsions, de nanoémulsions ou de microémulsions.

Les organopolysiloxanes sont définis plus en détail dans l'ouvrage de Walter NOLL « Chemistry and Technology of Silicones » (1968) Academie Press. Elles peuvent être volatiles ou non volatiles.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25 °C et de préférence 1.10⁻⁵ à 1 m²/s. La viscosité des silicones est, par exemple, mesurée à 25 °C selon la norme ASTM 445 Appendice C.

Parmi ces polyalkylsiloxanes, on peut citer à titre non limitatif les produits commerciaux suivants :
- les huiles SILBIONE^{®} des séries 47 et 70 047 ou les huiles MIRASIL^{®} commercialisées par RHODIA telles que, par exemple, l'huile 70 047 V 500 000 ;
- les huiles de la série MIRASIL^{®} commercialisées par la société RHODIA ;
- les huiles de la série 200 de la société DOW CORNING telles que plus particulièrement la DC200 de viscosité 60 000 mm²/s ;
- les huiles VISCASIL^{®} de GENERAL ELECTRIC et certaines huiles des séries SF (SF 96, SF 18) de GENERAL ELECTRIC.

On peut également citer les polydiméthylsiloxanes à groupements terminaux diméthylsilanol, connus sous le nom de dimethiconol (CTFA), tels que les huiles de la série 48 de la société RHODIA.

Dans cette classe de polyalkylsiloxanes, on peut également citer les produits commercialisés sous les dénominations « ABIL WAX^{®} 9800 et 9801 » par la société GOLDSCHMIDT qui sont des polyalkyl(C₁-C₂₀)-siloxanes.

Il peut s'agir de polyalkylarylsiloxanes, notamment choisis parmi les polydiméthyl/méthylphénylsiloxanes, les polydiméthyldiphényl-siloxanes linéaires et/ou ramifiés de viscosité de 1.10-⁵ à 5.10-² m²/s à 25 °C.

Parmi ces polyalkylarylsiloxanes on peut citer, à titre d'exemple, les produits commercialisés sous les dénominations suivantes :
- les huiles SILBIONE^{®} de la série 70 641 de RHODIA ;
- les huiles des séries RHODORSIL^{®} 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Des produits plus particulièrement utilisables conformément à l'invention sont des mélanges tels que :
- les mélanges formés à partir d'un polydiméthylsiloxane hydroxylé en bout de chaîne ou diméthiconol (CTFA) et d'un poly-diméthylsiloxane cyclique également appelé cyclométhicone (CTFA) tel que le produit Q2 1401 commercialisé par la société DOW CORNING ;
- les mélanges formés à partir d'une gomme polydiméthyl-siloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10-⁶ m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :
R₂SiO_{2/2}, R₃SiO_{1/2}, RSiO_{3/2} et SiO_{4/2} dans lesquels R représente un groupement hydrocarboné possédant 1 à 16 atomes de carbone ou un groupement phényle. Parmi ces produits, ceux particulièrement préférés sont ceux dans lesquels R désigne un groupe alkyle inférieur en C₁-C₄, plus particulièrement méthyle, ou un groupe phényle.

On peut citer parmi ces résines le produit commercialisé sous la dénomination « DOW CORNING 593 » ou ceux commercialisés sous les dénominations « SILICONE FLUID SS 4230 et SS 4267 » par la société GENERAL ELECTRIC et qui sont des silicones de structure diméthyl/triméthylsiloxane.

On peut également citer les résines du type triméthylsiloxysilicate commercialisées notamment sous les dénominations X22-4914, X21-5034 et X21-5037 par la société SHIN-ETSU.

Les silicones telles que décrites ci-dessus peuvent être utilisées seules ou en mélange, en une quantité comprise entre 0,01 et 20 % en poids, de préférence entre 0,1 et 5 % en poids par rapport au poids total de la composition.

Bien entendu, l'homme de l'art veillera à choisir ce ou ces éventuels composés complémentaires et/ou leurs quantités de manière telle que les propriétés avantageuses attachées intrinsèquement à l'association conforme à l'invention ne soient pas, ou substantiellement pas, altérées par la ou les adjonctions envisagées.

Ces compositions conviennent principalement au soin et/ou au conditionnement des cheveux.

Selon un mode de réalisation préféré de l'invention, la composition peut être utilisée comme après-shampooing.

Lorsque les compositions conformes à l'invention sont mises en oeuvre comme des après-shampooings classiques, elles sont simplement appliquées sur les cheveux généralement préalablement mouillés.

Les compositions selon l'invention peuvent être préparées par mélange des différents constituants à température ambiante et soumission de l'ensemble à un fort cisaillement.

Selon un premier mode de réalisation, l'ensemble des constituants de la composition est mélangé préalablement à l'opération de cisaillement.

Selon un autre mode de réalisation, l'ensemble des composants, à l'exception des fibres, est homogénéisé au préalable, avant ajout des fibres ou fibres préalablement gonflées à partir de la formulation. Il est également possible de procéder au mélange des composés sous chauffage.

Les exemples suivants sont donnés à titre illustratif et non limitatif de la présente invention.

### EXEMPLES

| Nom | Concentration (% massique) | |
|---|---|---|
| | Exemple 1 | Exemple 2 |
| Cetearyl alcool | 2,5 | - |
| Cetyl esters | 0,5 | - |
| Chlorure de Behentrimonium ² | 1,2* | - |
| Amodimethicone (et) tricedeth-12 (et) chlorure de cetrimonium ³ | 1,05* | - |
| Inuline (Chicorée) ⁴ | - | 15 |
| Polyquatemium-37 (et) huile minérale (et) PPG-1 trideceth-6⁵ | - | 0,25* |
| sel de Na de Copolymère acrylate réticulé ⁸ | 8 | 5 |
| Conservateurs | qs | qs |
| Parfum | qs | - |
| Agent de pH | qs | qs |
| Eau qsp | 100 g | 100 g |

| | | |
|---|---|---|
| les concentrations sont exprimées en % massique de matière active. ¹: Spermwax vegetal - ROBECO ²: solution hydroalcoolique à 80 % en MA - Genamin KDMP - CLARIANT ³ : Amodiméthicone en emulsion cationique à 35 % en matière active - DC 939 de DOW CORNING ⁴ : Raftiline HP - ORAFTI ⁵ : Emulsion inverse dans l'huile minérale à 50 % en MA - Salcare SC 95-CIBA ⁸: Oasis 121/6/10 - TECHNICAL ABSORBENTS (longueur 6 mm) | | |

Les fibres de polymère superabsorbant sont mises à gonfler dans une partie de l'eau de la composition, puis cette dispersion est introduite dans le mélange constitué par le reliquat de la formule, hormis le parfum. Le tout est porté à la température de 60 °C et est soumis à une forte agitation. Après dissolution des composés hydrosolubles et homogénéisation du milieu, on laisse refroidir sous agitation.

A 30 °C, on ajoute le parfum, puis on laisse refroidir à température ambiante. On obtient un après-shampooing d'aspect fibreux qui, appliqué sur cheveux, possède de bonnes propriétés d'application et de soin.

## Revendications

1. Composition de soin et/ou de conditionnement, notamment des matières kératiniques comprenant, dans un milieu physiologiquement acceptable, au moins :
- un polymère superabsorbant présent dans la composition sous forme de fibre, et
- un agent conditionneur cationique.

2. Composition selon la revendication précédente, comprenant de 1 à 50 %, voire de 5 à 25 % en poids à l'état sec du polymère par rapport à son poids total.

3. Composition selon l'une quelconque des revendications précédentes, dans laquelle les fibres de polymère(s) ont à l'état sec une longueur moyenne en nombre supérieure ou égale à 0,5 mm.

4. Composition selon la revendication précédente, dans laquelle lesdites fibres de polymère(s) possèdent à l'état sec une longueur moyenne en nombre supérieure
ou égale à 1 mm, notamment allant de 1 à 100 mm, en particulier de 2 à 80 mm, voire de de 3 à 60 mm, et mieux variant de 3 à 10 mm.

5. Composition selon l'une quelconque des revendications précédentes, dans laquelle ledit polymère superabsorbant est apte, lorsqu'il est à l'état sec, à absorber au moins 20 fois son poids en eau.

6. Composition selon l'une quelconque des revendications précédentes, dans laquelle ledit polymère résulte de la polymérisation avec réticulation partielle de monomère à insaturation éthylénique hydrosolubles, comme les polymères acryliques ou vinyliques.

7. Composition selon la revendication précédente, dans laquelle ledit polymère est un polyacrylate réticulé.

8. Composition selon l'une quelconque des revendications précédentes, comprenant plus de 0,1 %, notamment de 0,2 à 10 %, en particulier de 0,3 à 8 %, voire de 0,4 à 6 % en poids en agent(s) conditionneur(s) cationique(s).

9. Composition selon l'une quelconque des revendications précédentes, dans laquelle ledit agent est choisi parmi les polymères cationiques, les tensioactifs cationiques, les protéines cationiques, les silicones aminées et/ou leurs mélanges.

10. Composition selon la revendication précédente, comprenant un polymère cationique.

11. Composition selon la revendication précédente, dans laquelle le polymère cationique est choisi parmi (1) les homopolymères ou copolymères dérivés d'esters ou d'amides acryliques ou méthacryliques, (2) Les polysaccharides cationiques, (3) les polymères constitués de motifs pipérazinyle et de radicaux divalents alkylène ou hydroxyalkylène, (4) les polyaminoamides solubles dans l'eau, (5) les dérivés de polyaminoamides résultant de la condensation de polyalcoylènes polyamines avec des acides polycarboxyliques suivie d'une alcoylation par des agents bifonctionnels, (6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone, (7) les co-cyclopolymères d'alkyl diallyl amine ou de dialkyl diallyl ammonium, (8) les polymères de diammonium quaternaire contenant des motifs récurrents répondant à la formule : 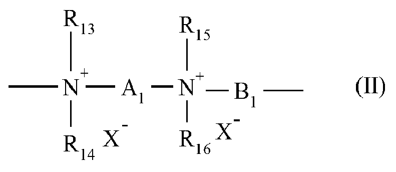 Formule (II) dans laquelle :
R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C₁-C₆ linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
A₁ et B₁ représentent des groupements polyméthyléniques contenant de 2 à 20 atomes de carbone, et
X- désigne un anion dérivé d'un acide minéral ou organique;
(9) les polymères de polyammonium quaternaires comprenant des motifs de formule (III): 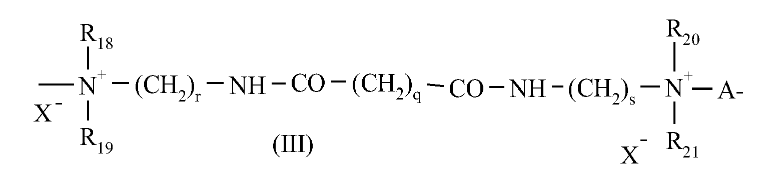 Formule dans laquelle :
R₁₈, R₁₉, R₂₀ et R₂₁, identiques ou différents, représentent un atome d'hydrogène ou un radical méthyle, éthyle, propyle, (β-hydroxyéthyle, (β-hydroxypropyle ou -CH₂CH₂ (OCH₂CH₂)ₚOH,
Où p est égal à 0 ou à un nombre entier compris entre 1 et 6, sous réserve que R₁₈, R₁₉, R₂₀ et R₂₁ ne représentent pas simultanément un atome d'hydrogène,
r et s, identiques ou différents, sont des nombres entiers compris entre 1 et 6,
q est égal à 0 ou à un nombre entier compris entre 1 et 34,
X- désigne un anion tel qu'un halogènure,
A désigne un radical d'un dihalogénure ou représente de préférence -CH₂-CH₂-O-CH₂-CH₂- ;
(10) les polymères quaternaires de vinylpyrrolidone et de vinylimidazole et (11) les polymères réticulés de sels de méthacryloyloxyalkyl(C₁-C₄) trialkyl(C₁-C₄)ammonium.

12. Composition selon la revendication 9, comprenant un tensioactif cationique.

13. Composition selon la revendication précédente, dans laquelle le tensioactif cationique est choisi parmi les sels d'amines grasses primaires, secondaires ou tertiaires, éventuellement polyoxyalkylénées ; les sels d'ammonium quaternaire ; les dérivés d'imidazoline ; les oxydes d'amines à caractère cationique, et/ou l'un de leurs mélanges.

14. Composition selon la revendication 9, comprenant une protéine cationique ou un hydrolysat de protéine cationique.

15. Composition selon la revendication 9, dans laquelle la silicone aminée est une silicone à groupements ammonium quaternaire.

16. Composition selon la revendication précédente, dans laquelle ladite silicone à groupements ammonium quaternaire est choisie parmi les silicones correspondant à une des formules générales suivantes : 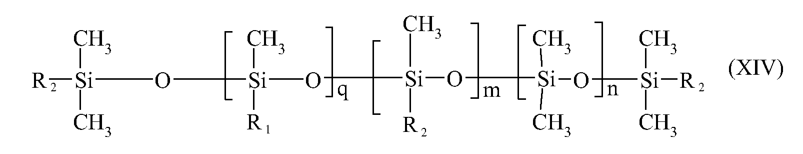 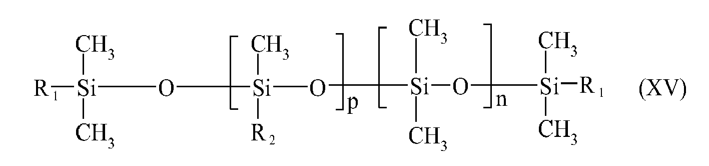 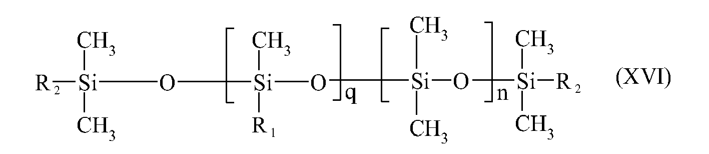 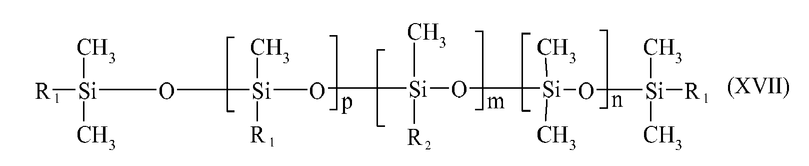 formules dans lesquelles :
- R₁, identique ou différent, représente un groupe alkyle, linéaire ou ramifié, en C₁-C₃₀ ou phényle ;
- R₂, identique ou différent, représente -C_{c}H_{2c}-O-(C₂H₄O)ₐ-(C₃H₆O)_{b}-R₅ ou
- C_{c}H_{2c}-O-(C₄H₈O)ₐ-R₅ ;
- R₃ et R₄, identiques ou différents, désignent chacun un groupe alkyle, linéaire
ou ramifié, en C₁-C₁₂, et de préférence un groupe méthyle ;
R₅, identique ou différent, est choisi parmi les groupes de formule suivante : 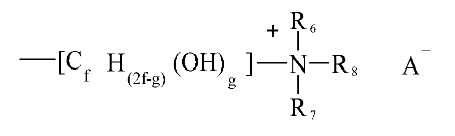 les radicaux R₈ représentent indépendamment un radical alkyle en C₁₋₂₂ ou alcényle en C₂₋₂₂, linéaire ou ramifié, et portant éventuellement un ou plusieurs groupements OH, ou représentent un groupement CₕH₂ₕZCOR₉ ;
- R₆, R₇ et R₉, identiques ou différents, représentent des radicaux alkyle en C₁₋₂₂ ou alcényle en C₂₋₂₂, linéaires ou ramifiés, portant éventuellement un ou plusieurs groupements OH, ou R₇ peut former avec une partie de R₈ un hétérocycle (cycle avec au moins un hétéroatome tel que par exemple N, O, P) ;
- m varie de 0 à 20,
- n varie de 0 à 500,
- p varie de 1 à 50,
- q varie de 0 à 20,
- a varie de 0 à 50,
- b varie de 0 à 50,
- c varie de 0 à 4,
- f varie de 0 à 4,
- g varie de 0 à 2,
- h varie de 1 à 4,
- Z représente un atome d'oxygène ou NH, et
- A- représente un anion minéral ou organique monovalent tel qu'un halogénure (chlorure, bromure), un sulfate, ou un carboxylate.

17. Composition selon l'une quelconque des revendications précédentes, dans laquelle la viscosité de la composition est supérieure à 1 Pa.s à 25 °C à un taux de cisaillement de 1s⁻¹.

18. Composition selon l'une quelconque des revendications précédentes, comprenant en outre des particules insolubles différentes de fibres de polymère superabsorbant.

19. Composition selon la revendication précédente, comprenant de 0,001 % à 20 % en poids, de préférence de 0,01 % à 10 % en poids et plus particulièrement de 0,1 à 3 % en poids de particules insolubles.

20. Composition selon l'une quelconque des revendications 18 et 19, dans laquelle les particules sont minérales.

21. Composition selon la revendication précédente, dans laquelle les particules minérales comprennent un élément des colonnes IIa, IIIa, IVa, IIIb et IVb du tableau de classification périodique des éléments et de préférence des colonnes IIa, IIIa et IVa.

22. Composition selon la revendication précédente, dans laquelle les particules sont choisies parmi les particules contenant au moins 10 % en poids de carbonate de calcium ou d'au moins un silicate tel que le talc, les particules contenant au moins 90 % d'oxyde d'aluminium, les silices, l'oxyde de magnésium, les oxydes de zinc, les oxydes de titane, le sulfate de baryum, le nitrure de Bore et leurs mélanges.

23. Composition selon l'une quelconque des revendications 18 à 22, dans laquelle les particules sont sous forme de plaquettes.

24. Composition selon l'une quelconque des revendications précédentes, associant des fibres de polymère acrylate réticulé à des plaquettes de talc et/ou de nitrure de Bore.

25. Composition selon l'une quelconque des revendications précédentes, comprenant de l'eau, notamment plus de 30 %, voire de 50 % à 95 % en poids d'eau par rapport au poids total de la composition.

26. Composition selon l'une quelconque des revendications précédentes, étant un après-shampooing.

27. Utilisation de fibres telles que définies dans l'une quelconque des revendications 1 à 7 pour la préparation d'une composition de soin et/ou de conditionnement des fibres kératiniques, notamment un après-shampooing.

28. Procédé de traitement cosmétique des matières kératiniques qui comprend l'application d'une quantité efficace d'une composition telle que définie dans l'une quelconque des revendications 1 à 26 sur les matières kératiniques et la réalisation d'un éventuel rinçage après un éventuel temps de pose.
